(19) Europäisches Patentamt
European Patent Office
Office européen des brevets

(11) Publication number: 0 129 667 A1

# (12) EUROPEAN PATENT APPLICATION

(21) Application number: 84104618.8

(22) Date of filing: 25.04.84

(51) Int. Cl.[4]: A 61 K 31/35

(30) Priority: 26.04.83 JP 74575/83

(43) Date of publication of application: 02.01.85 Bulletin 85/1

(84) Designated Contracting States: BE CH DE FR GB LI NL SE

(71) Applicant: Takeda Chemical Industries, Ltd.
27, Doshomachi 2-chome Higashi-ku
Osaka-shi Osaka, 541(JP)

(72) Inventor: Yamazaki, Iwao
9-21, Hibarigaokayamate 1-chome
Takarazuka Hyogo 665(JP)

(72) Inventor: Sawa, Yoichi
19-11, Sengokuhigashi-machi
Kadoma Osaka 571(JP)

(74) Representative: von Kreisler, Alek, Dipl.-Chem. et al,
Deichmannhaus am Hauptbahnhof
D-5000 Köln 1(DE)

(54) Compounds and pharmaceutical compositions for treatment of hypoovarianism.

(57) A compound of the general formula

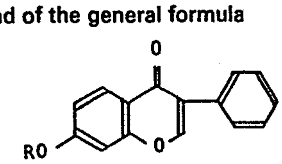

[wherein R is a hydrogen atom or a lower alkyl group] is effective for treatment of hypoovarianism.

EP 0 129 667 A1

TITLE MODIFIED
see front page



0129667

Method for Treatment of Hypoovarianism

This invention relates to a therapeutic means for treatment of hypoovarianism.

More particularly, this invention relates to a medicament containing a compound of the general formula

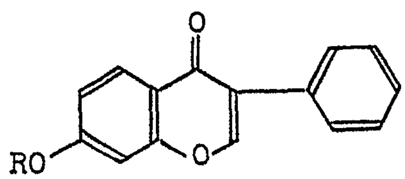

[wherein R is a hydrogen atom or a lower alkyl group]

for treatment of hypoovarianism. Hypoovarianism such as climacteric disturbances, infertility, etc. is caused by a decreased secretion of estrogen [hereinafter referred to as E] due to hypofunction of the ovary.

Climacteric disturbances are the syndrome which develop in females in climacterium (approximately at the ages of 45 to 60) as a decreased secretion of E results in changes in various metabolisms of the body which, in turn, affect the diencephalon which is the autonomic nerve center. Manifestations of climacteric disturbances are diverse and include angioneurotic disorder-like symptoms such as heat sensation, feeling of cold, hot flash, palpitation, etc., psychoneurotic disorder-like symptoms such as headache, dizziness, insomnia, etc.; perception disorder symptoms such as numbness, hyperesthesia, hypesthesia, etc.; locomotorial disorder symptoms such as lumbago, shoulder stiffness, arthralgia, etc.; cutaneous

secretory disturbance symptoms such as perspiration, dry mouth, etc.; obesity, emaciation, climacteric diabetes, senile vaginitis, pruritus cutaneus, fatigue, arteriosclerosis, cardiac disorder and so on. For the treatment of these manifestations of climacteric disturbance, therapies using a synthetic E drug or an androgen drug or both of them so as to correct for the decrease in secretion of endogenous ovarian hormone proved successful and were used as therapies of choice against climacteric disturbances. In recent years, however, these therapies have not been used as often as in the past because they present the risk of metrorrhagia and carcinogenicity. As other therapies, minor tranquilizers, antidepressants, autonomic blocking agents, peripheral circulation improving agents, chinese medicines, etc. have been administered but their efficacies are not satisfactory as are desired.

Infertility, which is said to be present in 5 to 10 percent of all the females of fertile ages (approximately up to the early forties), is caused by various causes and includes such cases due to insufficient E secretion as ovarian aplasia, ovulation disorder, uterine aplasia, nidation disorder, early abortion, etc. Against these types of infertility, synthetic E drugs and progesterones have been mainly employed. However, these drugs have the disadvantage that responses vary a great deal according to individuals.

The present inventors found that the compound of general formula (I)

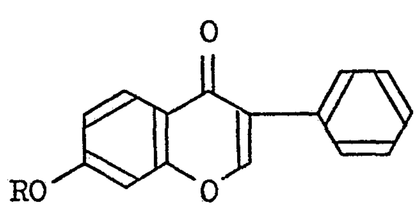

(I)

[hereinafter there may be occasionally referred to as the Compound (I) and wherein R is a hydrogen atom or a lower alkyl group] does not independently have E activity but does potentiate E activity and demonstrated that the compound potentiates E activity of ovarian origin which has been depressed in patients with climacteric disturbances or infertility, thus being useful for the treatment of these diseases.

Thus, the principal object of this invention is to provide a method for treatment of hypoovarianism through the mechanims of potentating the activity of E produced endogenously in the human ovary or administered exogenously, which comprises administering to the female the compound of general formula (I). Another object of this invention is to provide a pharmaceutical composition comprising the compound of general formula (I), which is usable in the above-mentioned method.

Referring, now, to the above general formula (I), the lower alkyl group R may be a straight-chain group or a branched-chain group, and may for example be methyl, ethyl, propyl, butyl or pentyl. The compound of general formula (I) can be produced, for example by cyclizing a 2-hydroxy-4-RO-substituted phenyl benzyl ketone in the conventional manner and the compound (I) wherein R is a lower alkyl group can also be produced by alkylating a 7-hydroxy-isoflavone.

The compound of general formula (I) which is employed in accordance with this invention is invariably a white to pale yellowish brown crystalline compound which is freely soluble in dimethylformamide and chloroform, soluble in ethanol and acetone, and practically insoluble in water.

As will be apparent from acute toxicity text shown hereinafter, the compound of general formula (I) did not cause death nor toxic symptoms attributable thereto when its technically feasible maximum dose (5,000 to 10,000 mg/kg) was administered orally or subcutaneously to mice and rats. Thus, the compound of general formula (I) is only sparingly toxic.

**Methods for the preparation of compound (I) are described in the prior art and some species of compound (I) and related isoflavones are known to be useful as agents for** increasing capillary strength (French Pharmaceutical Patent No. 1065), therapeutic drugs for vascular disorder, inflammation and P-hypovitaminosis conditions (United States Patent No. 3352754), body weight increasing agents (United States Patent Nos. 3864362, 4166862 and 4117149), agents for increasing muscle power, anticatabolic agents and antasthenics (United States Patent No. 3864362), therapeutic drugs for osteoporosis of immobilisation (United States Patent No. 3907830), and therapeutic drugs for myocardial or pulmonary insufficiency (Japanese Toku-Kai-Sho 53-133635), etc. However, nothing is known about the use of the compound as a therapeutic drug for hypoovarianism.

The dosage of the compound of general formula (I) according to this invention for humans is generally about 5 to 10 mg/kg/day for oral administration, and about 200 to 600 mg can be orally taken daily, once a day or, if necessary, in 2 to 3 divided doses. The compound (I) is preferably formulated into such dosage forms as tablets, capsules, etc. by the established pharmaceutical procedure. Such tablets and capsules can be prepared using suitable vehicle such as lactose, starch, etc., binders such as hydroxypropylcellulose, and lubricants such as magnesium stearate. The tablets may be sugar-coated, if necessary.

 

The following experimental examples show the activity of the compound of general formula (I) to potentiate E activity. In these examples, the species of the compound of general formula (I) used were 7-hydroxy-isoflavone [hereinafter referred to as Compound A] and 7-isopropyloxy-isoflavone [briefly, Compound B].

Test Example 1

E activity-potentiating effect of 7-hydroxy-isoflavone in young oophorectomized rats

Spraque-Dawley rats, 33 days of age and 11 days after oophorectomy for elimination of endogenous E activity, were assigned to groups of 5 to 7 individuals and treated subcutaneously with solutions of esterone in sesame oil or orally with suspensions of Compound A in 1% hydroxypropylcellulose solution, either independently or in combination, for 3 days. On the 4th day, each animal was autopsied and its uterine wet weight was recorded. As shown in Table 1, Compound A at 50 and 100 mg/kg did not cause an increase of uterine weight but potentiated the uterine weight increasing action of estrone.

Table 1

| Daily dose of estrone (μg/kg) | Daily dose of Compound A (mg/kg) | No. of animals | Uterine wet weight (mg±S.D.) |
|---|---|---|---|
| 0 | 0 | 5 | 37.4 ± 0.6 |
| 0 | 50 | 7 | 35.3 ± 1.7 |
| 0 | 100 | 7 | 35.9 ± 1.0 |
| 1.0 | 0 | 6 | 52.6 ± 2.3 |
| 1.0 | 50 | 6 | 60.8 ± 4.1 |
| 1.0 | 100 | 6 | 70.6 ± 2.7* |

*: Significant as compared with estrone 1.0 μg/kg group ($P < 0.01$).

Test Example 2

E activity-potentiating effect of 7-isopropyloxy-isoflavone in young oophorectomized rats

Spraque-Dawley rats, 33 days of age and 11 days after oophorectomy for elimination of endogenous E activity, were assigned to groups of 5 to 7 individuals and treated subcutaneously with solutions of esterone in sesame oil or orally with suspensions of Compound B in 1% hydroxypropylcellulose solution, either independently or in combination, for 3 days. On the 4th day, each animal was autopsied and its uterine wet weight was recorded. As shown in Table 2, Compound B at 50 and 200 mg/kg did not cause an increase of uterine weight but potentiated the uterine weight increasing action of estrone.

Table 2

| Daily dose of estrone (μg/kg) | Daily dose of Compound B (mg/kg) | No. of animals | Uterine wet weight (mg ± S.D.) |
|---|---|---|---|
| 0 | 0 | 7 | 29.2 ± 0.9 |
| 0 | 50 | 7 | 32.2 ± 1.3 |
| 0 | 200 | 7 | 34.7 ± 1.2 |
| 0.5 | 0 | 7 | 36.3 ± 1.9 |
| 0.5 | 50 | 7 | 39.6 ± 2.4 |
| 0.5 | 200 | 7 | 51.3 ± 5.3* |
| 1.0 | 0 | 7 | 51.9 ± 4.2 |
| 1.0 | 50 | 7 | 58.2 ± 2.6 |
| 1.0 | 200 | 7 | 71.5 ± 7.4$^{+}$ |

*: Significant as compared with estrone 0.5 μg/kg group ($P < 0.05$).

+: Significant as compared with estrone 1.0 μg/kg group ($P < 0.05$)

Acute toxicity

Five-week-old ICR mice and 5-week-old Spraque-Dawley rats were used in groups of 10 males and 10 females, and suspensions of compound A or B in olive oil were administered orally [2,500, 5,000 and 10,000 mg/kg of each compound] or subcutaneously (1,250, 2,500 and 5,000 mg/kg). The animals were kept under observation for 14 days. None of the groups showed deaths nor toxic symptoms which might be attributable to compound A or B, with the result that $LD_{50}$ could not be calculated.

The following preparation examples are given to illustrate the invention in further detail only and not to limit the scope of the invention thereto.

Preparation Example

1. Tablets

| | | |
|---|---|---|
| I) | 7-Isopropyloxy-isoflavone | 200 g |
| II) | Lactose | 15 g |
| III) | Starch | 44 g |
| IV) | ECG-500 | 10 g |
| V) | Magnesium stearate | 1 g |

The above components I) through V) were admixed to prepare 1000 uncoated tablets with a diameter of 8.5 mm.

2. Capsules

| | | |
|---|---|---|
| I) | 7-Hydroxy-isoflavone | 200 g |
| II) | Lactose | 40 g |
| III) | Starch | 50 g |
| IV) | Hydroxypropylcellulose | 7 g |
| V) | Magnesium stearate | 3 g |

The above components I) through V) were admixed and filled into 1000 No. 1 capsules.

What is claimed is:

1. A compound of the general formula

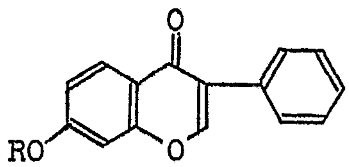

[wherein R is a hydrogen atom or a lower alkyl group] for use in treatment of hypoovarianism.

2. A pharmaceutical composition for treatment of hypoovarianism, which contains an effective amount of a compound of the general formula

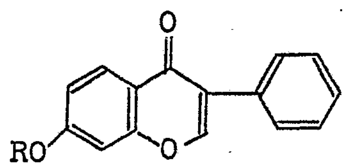

[wherein R is a hydrogen atom or a lower alkyl group] and a pharmaceutical acceptable carrier, vehicle, lubricant or diluent therefor.

3. A pharmaceutical composition according to claim 2, which is in the form of tablet, capsule, granule, fine granule, powder or syrup.

4. A pharmaceutical composition according to claim 2, wherein the hypoovarianism is climacteric disturbances or infertility.

European Patent Office

**EUROPEAN SEARCH REPORT**

0129667

Application number

EP 84104618.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,X | FR - M - 1 065 (LABORATORIES LAROCHE-NAVARRON)<br>* Claims 1,2,4,6 *<br>-- | 1-4 | A 61 K 31/35 |
| D,X | US - A - 3 352 754 (J.M. GAZAVE)<br>* Column 1, lines 50-67; column 4, lines 32-49; column 5, lines 8-16 *<br>-- | 1-4 | |
| X | DE - A - 2 166 458 (CHINOIN GYOGYSZER ES VEGYESZETI TERMEKEK GYARA RT)<br>* Page 5, line 26 - page 7, line 19 *<br>-- | 1-4 | |
| D,X | US - A - 3 907 830 (L. FEUER et al.)<br>* Column 7, line 58 - column 8, line 20; column 11, lines 24-52 *<br>-- | 1-4 | TECHNICAL FIELDS SEARCHED (Int. Cl. 3)<br>A 61 K 31/00<br>A 23 K 1/00 |
| D,X | US - A - 3 864 362 (L. FEUER et al.)<br>* Column 1, line 46 - column 2, line 45 *<br>---- | 1-4 | |

The present search report has been drawn up for all claims

| Place of search | Date of completion of the search | Examiner |
|---|---|---|
| VIENNA | 11-07-1984 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82